# EUROPEAN PATENT SPECIFICATION

(11) **EP 1 398 000 B1**
(45) Date of publication and mention of the grant of the patent: **09.08.2006**
(21) Application number: 02019635.8
(22) Date of filing: 03.09.2002
(51) Int. Cl.: A61B 17/72

(54) **Surgical nail and screw fixation system**
Nagel und Schraube für chirurgisches Fixationssystem
Broche et vis pour la fixation chirurgicale

(43) Date of publication of application: 17.03.2004
(73) Proprietor: Pennig, Dietmar, Prof. Dr., 50935 Köln (DE)
(72) Inventor: Pennig, Dietmar, Prof. Dr., 50935 Köln (DE)
(74) Representative: Botti, Mario

(56) References cited:
- DE-U- 8 907 443
- FR-A- 2 784 283
- US-A- 3 709 218
- US-B1- 6 355 043

## Description

### Technical Field

The present invention relates to a nail for treatment of bone fractures and, more specifically to a nail for improving the fixation of proximal fractures of the humerus bone.

The invention further relates to a system including at least a humeral nail to be inserted in a humeral shaft and comprising at least a proximal transversal hole for the passage of a corresponding locking screw, said screw having a screw head and a screw body.

### Background Art

As is well known in this specific orthopaedic field, proximal humeral fractures are the most common humeral fractures.

The humerus bone presents an upper part that may be considered a ball and socket joint at the shoulder. The proximal end of the humerus has an enlarged head including the ball portion. The humeral shaft extends distally away from the proximal end, toward the elbow joint.

Simple proximal humeral fractures present the humerus head or a portion of the head that is broken from the humeral shaft, often with minimal displacement.

More complex humeral fractures present two, three or four portions of the head that are broken with major displacement.

These displaced portions of the humerus head are called fragments.

The nature of the humeral fractures is generally predictable since the head tends to fracture between the ball portion and one or both tubercles of the head to which ligaments are attached. However, the reduction of a humeral fracture is still one of the most complex operation in the orthopaedic surgery.

According to the prior art the shoulder joint may be surgically replaced by a prosthesis; however, this solution may be unsatisfactory for the patient under many points of view.

More recently, new conservative surgical techniques are gaining more and more approval for the use of a humeral nail with locking screws.

The nail is designed to be inserted in the proximal end of the humerus while the reduction and consolidation of the fragments is obtained using screws passing through proximal holes in the nail.

A nail of this kind is disclosed for instance in the German patent application No. DE 199 45 611 relating to a humeral nail having a shaft with transverse holes to hold fixing screw. Several holes in the proximal section of the shaft are offset in their height and angles to allow the screws holding the fragments in different directions.

A similar solution is also disclosed in the US patent No. 5,472,444 relating to a humeral nail having transverse holes oriented at selected angles to receive screws attached to the fragments.

When using this kind of humeral nails and locking screws it has been noted that the threaded portion of the screws tend to loose stability and overall solidity over time. In other words, the threaded portion of a screw, implanted into a corresponding fragment, looses its fastening effect since the porous bone leaves the screw free to move and rotate within the transverse hole.

This problem is even more evident in proximal humeral fractures that are particularly problematic such as those involving elderly, osteoporotic patients or in patients having cancerous tissue in the regions of the fracture.

In order to solve this further problem a prior art solution has been recently provided by using a humeral nail including at least three proximal screwholes and fully-threaded screws suitable for cancellous fixation of small bone fragments.

The bone fragments are fixed against the nail and held in place by simple locking of the screws in the screwholes. Thus, the screws are stable in the nail.

While allowing a fast proximal reconstruction of the humeral fractures, this last prior art solution still presents a serious drawback due to the fact that, after the anatomical reduction of the fractures, at least one of the screws heads remains hidden by a bone fragment, generally the lesser tuberosity. Thus, a fast removal of this screw from the nail after consolidation is not possible and this limits a fast removal of the nail.

Moreover, even when using a fully-threaded screw the problem encountered with osteoporotic patients are not totally solved since the fragments are pulled toward the nail but inside a osteoporotic bone the threaded portion of the screw doesn't offer the required stability to forbid the rotation of the screw and its release from the corresponding screwhole.

A further drawback is that the screws cannot be inserted in any possible direction but are compelled to lie just in the direction transversal to the nail and that is not always the optimal direction to fasten all possible fragments.

A known prior art solution is disclosed in the French patent application No. FR 2 784 283 wherein in a solid humeral nail a special rib is provided transversally to a nail hole for engaging a screw when this screw is released from the bone.

The object of the present invention is that of providing a new fixation system for improving the fixation of proximal fractures of the humerus, even in osteoporotic bones, without the drawbacks affecting the prior art solutions and, more specifically, allowing to insert easily the screws through the corresponding nail holes even along different directions.

A further object of the present invention is that of allowing fixation and consolidation of proximal fractures of the humerus through fastening means that are easy to use during the surgery operation.

Another object of the present invention is that of reducing the stress on osteoporotic bone fragments while providing a stronger fastening action of such fragments.

Another object of the present invention in that of allowing the surgeon to fix together multiple fragments with a smaller incision of the muscle surrounding the bone.

### Disclosure of Invention

According to a first embodiment of the present invention a new nail and screw fixation system is provided including at least a humeral nail to be inserted in a humeral shaft and comprising at least a proximal transversal hole for the passage of a corresponding locking screw, said screw having a screw head and a screw body. Advantageously, said transversal hole has an internal partially threaded portion and the corresponding screw has an outside thread diameter smaller than the diameter of said at least one transversal hole that receives such a screw.

The partially threaded portion works as a portion of nut screw but doesn't interfere with the screw during the normal insertion. In case of a undesired movement or displacement of the screw after its final fastening or in use, the internal threaded portion solve the problem engaging the threaded portion of the screw at least in a crest to crest fashion.

This solution allows to keep in place and keep stable in the nail the screw, inside the corresponding hole, even when a movement or a release of the screw occurs in an osteoporotic bone.

Further features and advantages of the nail and screw system according to the present invention will appear from the following description of a preferred embodiment given by way of indicative and non-limiting example with the aid of the annexed drawings.

### Brief Description of Drawings

Figure 1 shows a perspective view of a humeral nail and corresponding locking screw of the inventive fixation system;
Figure 2 is an enlarged view of the profile of the threaded portion of the screw shown in Figure 1;
Figure 3 shows a front sectional view of a particular of the nail and screw system of Figure 1;
Figures 4 and 5 show side and a cross sectional views of the particular of Figure 3 in different working conditions, respectively;
Figure 6 is a further perspective view of the fixation system according to the present invention;
Figure 7 is a top view of the fixation system of Figure 6;
Figure 8 is a further perspective and partially sectional view of a further embodiment of the present invention;
Figure 9 is a top view of the fixation system of Figure 8;

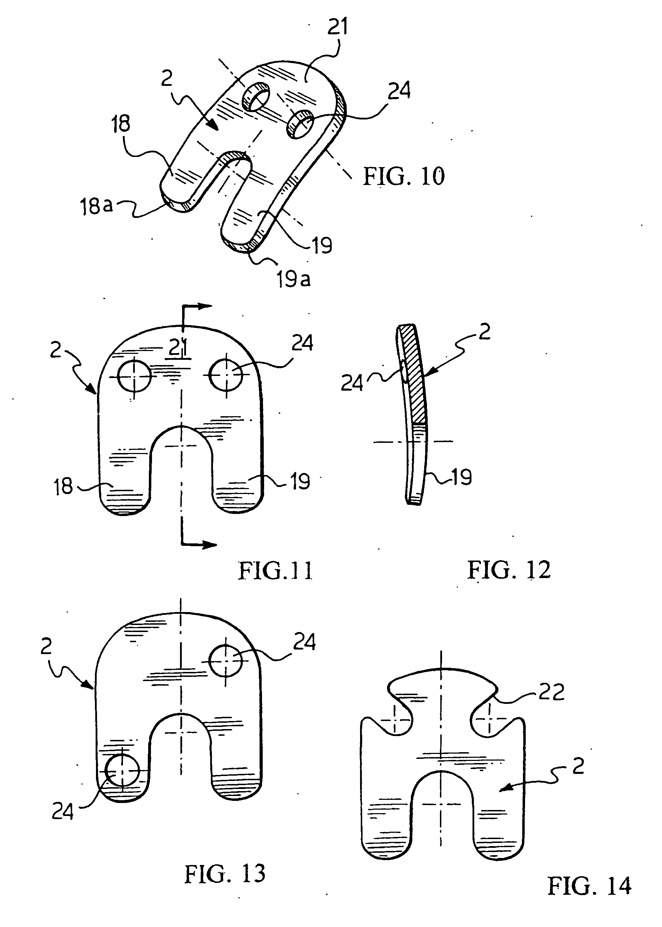

Figure 10 is a perspective view of particular fastening device to be used in the fixation system according to the present invention;
Figures 11 and 14 show front and side views of the fastening device of Figure 10, respectively;

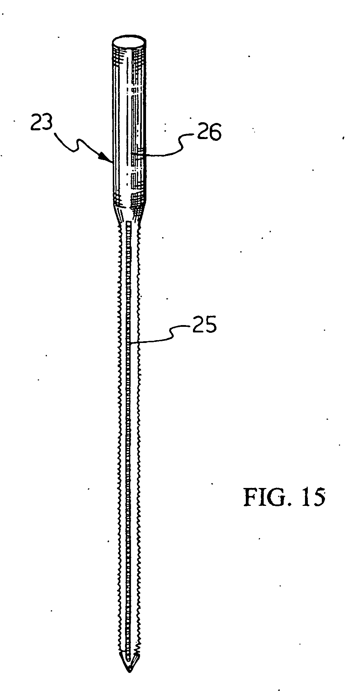

Figure 15 is a perspective view of a particular of the fixation system of Figures 6 and 8.

### Modes for Carrying Out the Invention

With reference to the drawings figures, a nail and screw system for improving the fixation of proximal fractures of the humerus is globally shown at 1.

The fixation system 1 of the present invention comprises a humeral nail 10 that is directly inserted in the humeral shaft 9. The nail 10 is cannulated and is inserted into the medullar bone cavity 8.

The nail 10 may be either cylindrical for all its length or have a conically tapered end portion. So, the nail 10 may have a constant or varying circular cross section throughout its length or may even present a curved portion; the form and length of the nail is not a limiting aspect of the inventive system.

The distal portion of the nail 10 presents a closed end 11 having a hemispherical form and forming a so-called "nose" of the nail.

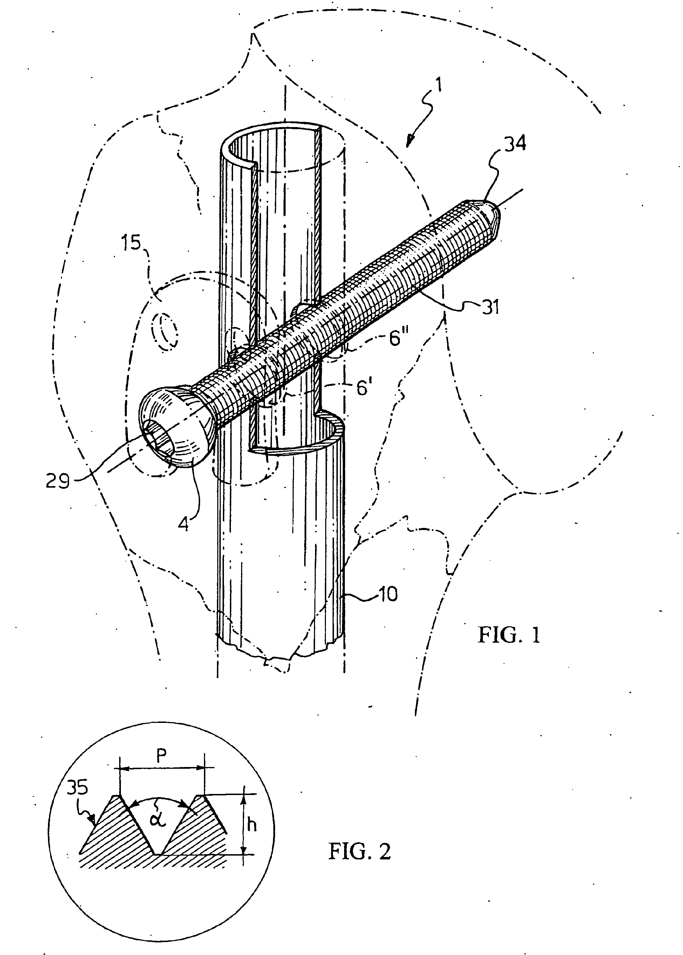

Distal and transverse holes are provided in the distal portion of the nail 10 to receive locking screws. For instance, as shown in Figure 1, a couple of holes 12, 13 receive corresponding locking screws 32, 33 for securing the nail 10 once is has been inserted into the humeral shaft 9.

According to a first embodiment of the present invention the fixation system 1 includes the humeral nail 10 and at least a proximal locking screw 3.

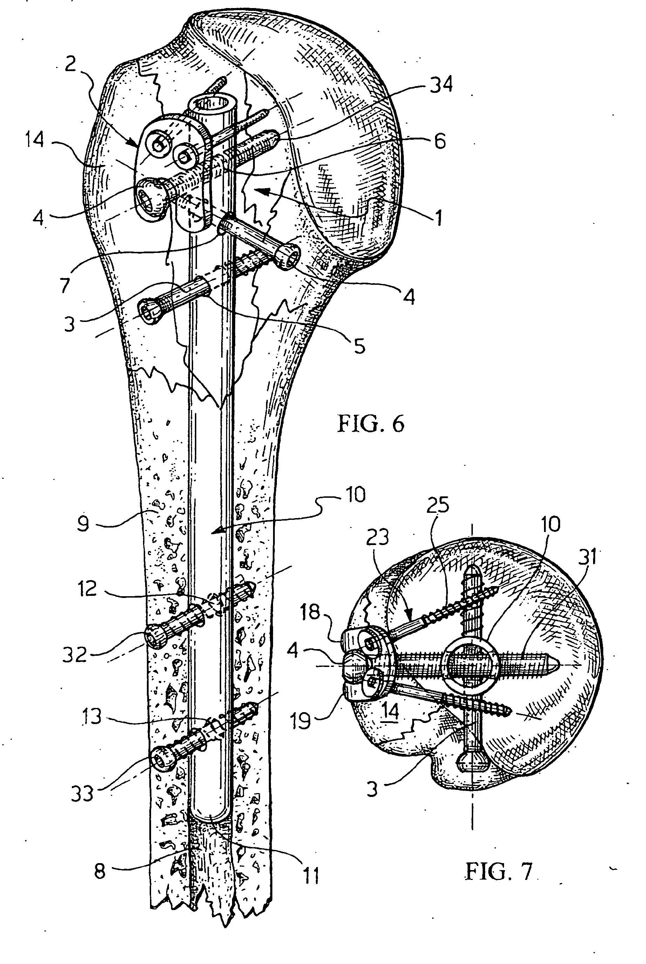

The humeral nail 10 may even include a group of proximal transversal holes for corresponding locking screws 3. For instance, in this embodiment at least three transversal holes 5, 6, 7 are shown in the proximal portion of the humeral nail 10, as shown in Figure 7.

More specifically, since the humeral nail 10 is cannulated, each of the transversal holes 5, 6, 7 comprises a couple of holes that are realised in opposite walls of the nail and that may be axially aligned. For disclosure purposes we will consider these opposite holes as a single transversal hole.

Each of the transverse holes may be oriented at a selected angle with respect to the nail longitudinal axis; however, at least two holes 5, 6 of said group of proximal holes lie on a same plane while a third hole lies in a sagittal plane.

Advantageously, the locking screws 3 are fully-threaded and present a working head 4, a screw body 31 and a rounded end 34, opposite to said working head. The working head 4 has a recessed shaped seat 29 to receive a working key, not shown and used by the surgeon.

The form of the screw's thread will be detailed disclosed hereinafter; however, it must be noted that any specific shape and thread of the screws 3 may be used in the fixation system 1 according to the invention.

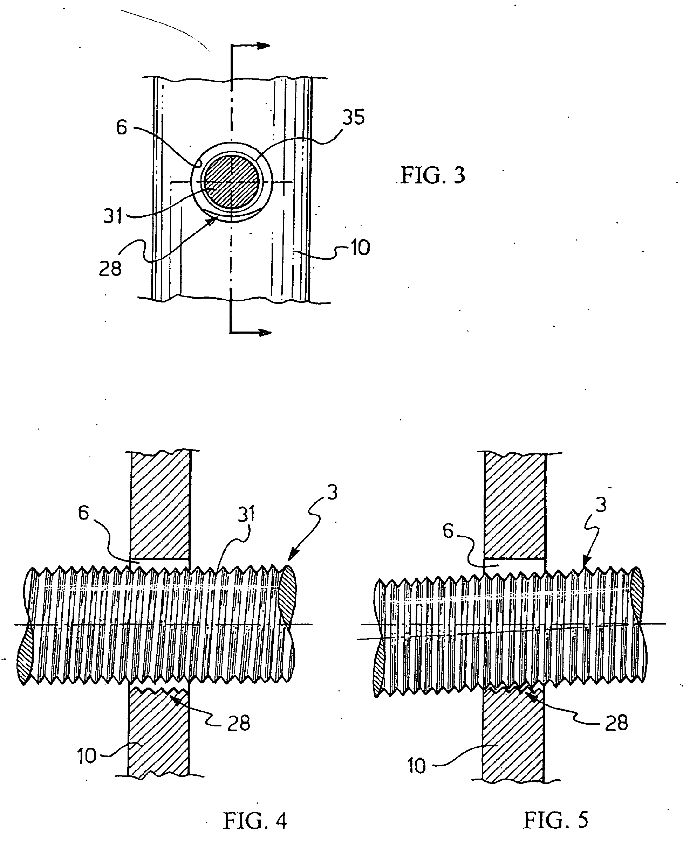

In this specific embodiment at least the proximal transversal hole 6 of the humeral nail 10 includes an internal partially threaded portion 28, clearly shown in Figures 4 and 5. This portion 28 may be considered a portion of nut screw, but even a knurl portion could meet the purposes of the invention.

As previously noted, the humeral nail is cannulated and each transversal hole comprises a couple of holes that are realised in opposite walls of the nail and that are axially aligned.

In this embodiment it's also important to identify the two opposite holes 6', 6" that form the transversal hole 6.

Each screw 3 has an outside thread diameter smaller than the diameter of the transversal hole 6 that receives the screw and the internal threaded portion 28 doesn't interfere with the screw during the normal insertion.

In a preferred embodiment the internal threaded portion 28 is provided in the hole 6' closer to the screw head 4.

The screw 3 may be put in place through the holes 6', 6" and screwed into the corresponding bone fragment to be fixed.

In case of a undesired movement or displacement of the screw 3 after its final fastening or in use, the internal threaded portion 28 solve the problem engaging the threaded portion of the screw 3 at least in a crest to crest fashion, as shown in Figure 4.

As previously mentioned, the screw 3 may have any specific shape and thread; however, a preferred screw structure is disclosed hereinafter.

The screw 3 comprises a screw body 31 that is fully threaded.

This threaded body 31 has a substantially cylindrical shape with a constant diameter ending in the rounded end 34.

Figure 6 is an enlarged view of the profile of the threaded portion according to the invention.

Advantageously, the threaded portion has at least a section formed with a constant pitch p, preferably a 0.45 mm pitch, and comprises threads 35 having a triangular profile in cross-section with a cusp or acute apex angle of 60°.

The facing walls of two adjacent threads form an angle α of no more than 60°.

Advantageously, the bottom portion of the threads between adjacent inner walls of the threads 35 is slightly concave and rounded, effective to ease the material flow and relieve bone stress during the screw penetration.

The radius of the bottom rounded portion is 0.5 mm.

The thread height h is 0.27 mm and it is constant along the thread profile.

This thread would allow also a conical profile to be used.

The screw 3 of this invention distributes the stress better than conventional screws. Moreover, the screw 3 of this invention shows to have a better distributed compression even in the absence of interference, that is in situations of an oversize pre-drilled hole, lysis or osteoporosis.

Referring now to Figures from 6 to 15 a further embodiment of the present invention is disclosed.

In this embodiment this system 1 includes also an inventive fastening device 2 for fixing the locking screw 3 or screws in their final fastening position.

As already mentioned, the locking screws 3 has a corresponding screw head 4 that, according to the prior art, would normally abut against the nail 10 or against the cortex 14 of the humeral bone.

On the contrary, according to the system of the present invention the fastening device 2 is interposed between the screw head 4 and the bone cortex surface 14. More specifically, said fastening device 2 comprises at least an intermediate plate element 15 that is inserted between said screw head 4 and the bone cortex surface 14.

Thus, when the screw 3 is totally fastened, the screw head 4 abuts against the intermediate plate element 15 instead of against the bone cortex 14.

This solution allows the enlargement of the abutting surface of the locking screw head 4 against the bone cortex surface 14, thus allowing a stronger fastening action and avoiding a further rotation of the screw 3. Moreover, this solution allows the surgeon to fix together multiple fragments with a smaller cut.

Advantageously, said intermediate plate element 15 includes a slightly curved surface 16 to adhere perfectly to the bone cortex 14 surface, as shown in Figures 5 and 7. In a preferred embodiment the plate element 15 is slightly bent in both main axial directions thus presenting two opposite and parallel curved surfaces 16 and 17.

Just to give some indications about the shape and dimension of the intermediate plate 15, it worth to note that the whole plate element 15 could fit inside a circle having a diameter of eighteen mm and the plate thickness is of about 1 mm.

The intermediate plate 15 and the screw 3 are made by surgical steel. As an alternative, they may be realised in Titanium or in a Titanium alloy.

As a possible alternative the plate 15 may be realised by a plastic material or biodegradable material.

Moreover, the intermediate plate element 15 has a substantially rounded profile and comprises a couple of elongated arm portions 18, 19 that are inserted in an astride position on the screw rod just before a final fastening action on the screw head 4.

Each of said arm portions 18, 19 presents a rounded end 18a, 19a.

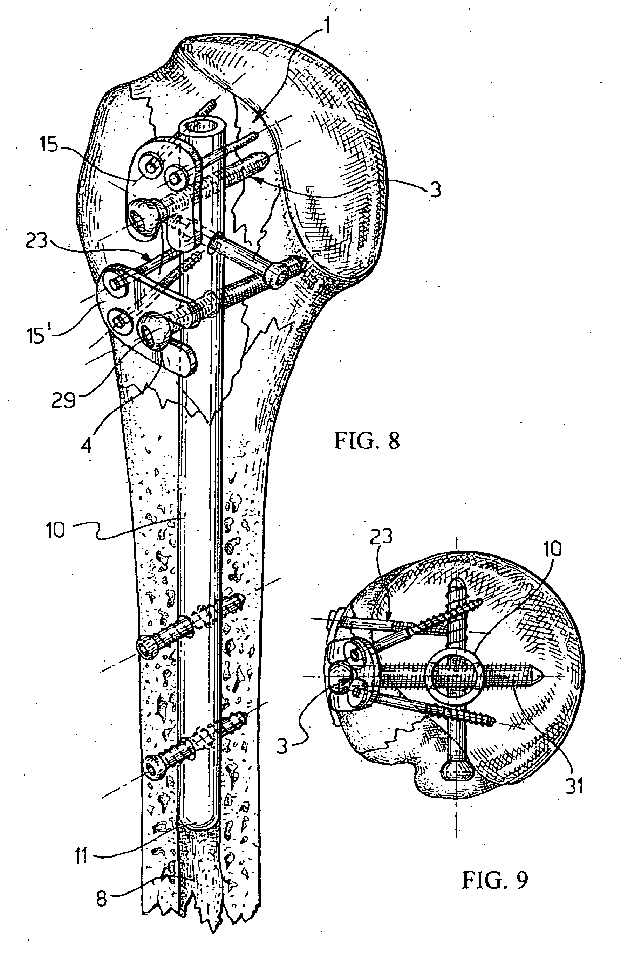

Said intermediate plate element 15 comprises an enlarged portion 21 having at least a seat 22, as shown in Figure 9, for embracing at least a fragment fixation pin 23 to be inserted into the bone, as shown in Figures 1 and 2.

In a preferred embodiment said seat 22 is a hole 24 formed in said enlarged portion 21 of the intermediate plate 15. This hole 24 may have for instance a diameter of 2,5 mm.

In a preferred embodiment, at least two holes 24 are provided in said enlarged portion 21 and in alignment with each corresponding arm portion 18, 19, as shown in Figure 6.

In a further embodiment of the present invention one of the holes 24 may be provided close to the end of one of the arm portions 18 or 19, as shown in Figure 8.

According to the previous description the intermediate plate element 15 may be considered an open washer integrally formed with a flange portion 21.

The fragment fixation pin 23 is a thin rod 26, made of surgical steel, having a final threaded portion 25. The rod may have a diameter of 3mm while the threaded portion of the rod may have an outside diameter less than 2,5 mm.

This fragment fixation pin is disclosed in the European Patent No. 0 642 323 in the name of the same applicant and shown schematically in Figure 5.

After the surgeon has produced an anterior opening in the deltoid muscle to reach the fractured epiphyseal mass, a short incision is made in line with the deltoid fibers. Then, the deltoid fibers are divided to access to the fractured area and to the corresponding ematoma.

The nail 10 is inserted into the axis of the humeral shaft 9 and kept in place by the distal screws 32, 33. Then the fractures of the humeral head may be reduced by fixing the fragments against the humeral nail 10.

Using specific tools, the bone fragments (lesser tuberosity, greater tuberosity, humeral head, etc...) once positioned, are precision drilled before receiving the screws 3.

The screws are locked and just before the final fastening of the screw the intermediate plate 15 between the screw head 4 and the bone cortex 14 in an astride position on the screw body. It worth to note that a very small incision of the deltoid muscle, for instance 4 cm, is required to insert the plate 15 and this aids healing.

When the intermediate plate element 15 is put into its final position and the screw 3 is fastened so that the screw head 4 abuts against the plate 15 then the fragment fixation pin 23 may be inserted in the bone through the hole 24.

The fragment fixation pin 23 may be inserted in the bone drilling a hole through the bone small enough to firmly engage the pin threads.

Advantageously, the fixation pins 23 may be oriented according to the needs of engagement of other possible fragments of the humeral head since the diameter of its threaded portion is a little smaller than the diameter of the hole 24.

The presence of the pin 23 has the double function to avoid displacement or rotation of the intermediate plate 15 and, in case of need, to fix a fragment of the humeral head.

According to a preferred embodiment of the invention a couple of fragment fixation pins 23 are inserted in the bone through the corresponding holes 24 of a plate 15 shaped as in the example of Figure 5 or 8.

However, as previously mentioned, even one fixation pin 23 may be enough for the purpose of avoiding a displacement of the plate 15 and a plate 15 including a single hole 24 may be used.

In a further embodiment of the present invention, disclosed with reference to Figures 3 and 4, a couple of intermediate plate elements 15 may be used for fixation purposes.

Those plates 15 may even have different dimension and a second plate 15' used in cooperation with the more distal of the proximal screws 3 may be greater than the other upper plate 15.

As a further alternative, the arm portions 18 and 19 of the second and more distal intermediate plate 15' may be longer than those shown in Figure 1 as a first embodiment of the present invention.

The final orientation of the plates 15 and 15' may be different, the surgeon just needs to pay attention to the position of the holes 24 so that the fragment fixation pins 23 don't interfere with the nail 10 hidden inside the bone shaft 9, as shown in Figures 3 and 4.

Even the position of the plates 15 and 15' may in some occasion be inverted, so that the larger plate 15' may be fixed by the corresponding screw 3 in the more proximal position.

As a whole the system according to the present invention facilitates a fast anatomical reduction of the fracture and ensured a fast consolidation as well.

Moreover, this system requires a very small incision to be made in the muscle surrounding the bone and this aids healing.

Moreover, the use of the plate 15 allows one to fix together multiple fragments with a smaller cut.

## Claims

1. Nail and screw system (1) for improving the fixation of proximal fractures of the humerus, including at least a cannulated humeral nail (10) to be inserted in a humeral shaft (9) and comprising at least a proximal transversal hole (6) for the passage of a corresponding locking screws (3), said screw (3) having a screw head (4) and a screw body (31); wherein said transversal hole (6) has an internal partially threaded portion (28) and the corresponding screw (3) has an outside thread diameter smaller than the diameter of said at least one transversal hole (6) that receives such a screw, said partially threaded portion (28) being a portion of nut screw or a knurl portion.

2. Nail and screw system according to claim 1, wherein said transversal hole (6) comprises a couple of opposite holes (6', 6") on opposite wall of the cannulated nail (10) and the hole (6') closer to the screw head (4) includes said partially threaded portion (28),

3. Nail and screw system according to claim 1, wherein said screw body (31) is fully threaded with a constant pitch (p) and comprises threads (35) having a triangular cross-section profile.

4. Nail and screw system according to claim 3, wherein said triangular cross-section profile has cusp or acute apex angles of 60°.

5. Nail and screw system according to claim 1, further including at least an intermediate plate element (15) inserted between said screw head (4) and the bone cortex surface (14) so that the head (4) is abutting against said plate (15).

6. System according to claim 5, wherein said intermediate plate element (15) includes a slightly curved surface (16) to adhere substantially to the bone cortex surface (14).

7. System according to claim 5, wherein said intermediate plate element (15) comprises a couple of elongated arm portions (18, 19) that are inserted in an astride position on the screw body before the final fastening of the screw head (4).

8. System according to claim 7, wherein said elongated arm portions (18, 19) present rounded ends.

9. System according to claim 5, wherein said intermediate plate element (15) comprises an enlarged portion (21) having at least a seat (22) for embracing at least a fragment fixation pin (23).

10. System according to claim 9, wherein said seat (22) is at least a hole (22) formed in said enlarged portion (21) of the intermediate plate element (15).

11. System according to claim 9, wherein said seat (22) is at least a hole (24) formed in at least one of said elongated arm portions (18, 19).

12. System according to claim 5, wherein said intermediate plate element (15) has a substantially rounded profile.

13. System according to claim 5, wherein said intermediate plate element (15) is an open washer integrally formed with a flange portion (21).

14. System according to claim 5, wherein a second intermediate plate element (15') is inserted between the screw head (4) of a second locking screw (3), passing through a second proximal hole (6) of the nail (10), and the bone cortex surface (14) .

15. System according to claim 14, wherein said second intermediate plate element (15') is larger than a first intermediate plate element (15).

16. System according to claim 14, wherein said second intermediate plate element (15) comprises a couple of elongated arm portions (18, 19) that are inserted in an astride position on the corresponding screw body.

## Patentansprüche

1. Nagel- und Schraubsystem (1) für die Verbesserung der Fixierung von proximalen Brüchen des Humerus, das mindestens einen rohrförmigen Humerusnagel (10) umfasst, der in einen Humerusschaft (9) einzufügen ist, und mindestens eine proximale transversale Öffnung (6) für den Durchgang einer entsprechenden Arretierschraube (3) aufweist, wobei die Schraube (3) einen Schraubenkopf (4) und einen Schraubenkörper (31) hat und die transversale Öffnung (6) ein inneres Teilgewindeteil (28) und die zugehörige Schraube (3) einen äußeren Gewindedurchmesser hat, der kleiner als der Durchmesser der mindestens einen transversalen Öffnung (6) ist, die diese Schraube aufnimmt, und wobei das Teilgewindeteil (28) ein Teil einer Schraubenmutter oder eines Rändelungsteils ist.

2. Nagel- und Schraubsystem nach Anspruch 1, wobei die transversale Öffnung (6) ein Paar von gegenüberliegenden Öffnungen (6', 6") in gegenüberliegenden Wänden des rohrförmigen Nagels (10) umfasst und die zu dem Schraubenkopf (4) näher liegende Öffnung (6') das Teilgewindeteil (28) umfasst.

3. Nagel- und Schraubsystem nach Anspruch 1, wobei der Schraubenkörper (31) vollständig mit einem Gewinde mit einer konstanten Steigung (p) versehen ist und ein Gewinde (35) mit einem dreieckigen Querschnittsprofil umfasst.

4. Nagel- und Schraubsystem nach Anspruch 3, wobei das dreieckige Querschnittsprofil spitze oder scharfe Öffnungswinkel von 60° hat.

5. Nagel- und Schraubsystem nach Anspruch 1, weiter umfassend mindestens ein Zwischenplattenelement (15), das zwischen dem Schraubenkopf (4) und der Knochenkortexoberfläche (14) eingefügt ist, so dass der Kopf (4) an der Platte (15) anliegt.

6. System nach Anspruch 5, wobei das Zwischenplattenelement (15) eine leicht gebogene Oberfläche (16) umfasst, um im Wesentlichen an der Knochenkortexoberfläche (14) festzuhaften.

7. System nach Anspruch 5, wobei das Zwischenplattenelement (15) ein Paar Längsarmteile (18, 19) umfasst, die in einer reitenden Position auf den Schraubenkörper vor dem endgültigen Befestigen des Schraubenkopfes (4) eingefügt sind.

8. System nach Anspruch 7, wobei die Längsarmteile (18, 19) abgerundete Enden aufweisen.

9. System nach Anspruch 5, wobei das Zwischenplattenelement (15) einen erweiterten Bereich (21) aufweist, der mindestens einen Sitz (22) zum Umarmen von mindestens einem Fragmentfixierungsstift (23) hat.

10. System nach Anspruch 9, wobei der Sitz (22) mindestens eine Öffnung (22) ist, die in dem erweiterten Bereich (21) des Zwischenplattenelementes (15) gebildet ist.

11. System nach Anspruch 9, wobei der Sitz (22) mindestens eine Öffnung (24) ist, die in mindestens einem der Längsarmteile (18, 19) gebildet ist.

12. System nach Anspruch 5, wobei das Zwischenplattenelement (15) im Wesentlichen ein abgerundetes Profil hat.

13. System nach Anspruch 5, wobei das Zwischenplattenelement (15) eine offene Unterlegscheibe mit it integral ausgebildeten Flanschteilen (21) ist.

14. System nach Anspruch 5, wobei ein zweites Zwischenplattenelement (15') zwischen dem Schraubenkopf (4) einer zweiten Verriegelungsschraube (3), die durch eine zweite proximale Öffnung (6) des Nagels (10) durchgeht, und der Knochenkortexoberfläche (14) eingefügt ist.

15. System nach Anspruch 14, wobei das zweite Zwischenplattenelement (15') größer ist als das erste Zwischenplattenelement (15).

16. System nach Anspruch 14, wobei das zweite Zwischenplattenelement (15') ein Paar Längsarmteile (18, 19) umfasst, die in einer reitenden Stellung an dem zugehörigen Schraubenkörper eingefügt sind.

## Revendications

1. Système à clou et vis (1) destiné à améliorer la fixation des fractures proximales de l'humérus, incluant au moins un clou canulé huméral (10) à insérer dans la diaphyse d'un humérus (9) et comprenant au moins un trou transversal proximal (6) pour le passage d'une vis de fixation correspondante (3), ladite vis (3) ayant une tête de vis (4) et un corps de vis (31), **caractérisé en ce que** ledit trou transversal (6) présente une partie interne partiellement filetée (28) et la vis correspondante (3) présente un diamètre de filetage externe inférieur au diamètre dudit au moins un trou transversal (6) qui reçoit une telle vis, ladite partie partiellement filetée (28) étant une partie de d'une vis à écrou ou une partie à molette.

2. Système à clou et vis selon la revendication 1, **caractérisé en ce que** ledit trou transversal (6) comprend une paire de trous opposés (6', 6") sur la paroi opposée du clou canulé (10) et **en ce que** le trou (6') le plus proche de la tête de vis (4) renferme ladite partie partiellement filetée (28) .

3. Système à clou et vis selon la revendication 1, **caractérisé en ce que** ledit corps de vis (31) est entièrement fileté avec un pas constant (p) et comprend un filetage (35) ayant un profil triangulaire en section transversale.

4. Système à clou et vis selon la revendication 3, **caractérisé en ce que** ledit profil triangulaire en section transversale présente des angles au sommet de 60° cuspides ou aigus.

5. Système à clou et vis selon la revendication 1, incluant de plus au moins un élément plan intermédiaire (15) inséré entre ladite tête de vis (4) et la surface du cortex de l'os (14) de façon à ce que la tête (4) vienne en butée contre ladite plaque (15).

6. Système selon la revendication 5, **caractérisé en ce que** ledit élément plan (15) inclut une surface légèrement courbe (16) de façon à adhérer en grande partie à la surface du cortex de l'os (14).

7. Système selon la revendication 5, **caractérisé en ce que** ledit élément plan intermédiaire (15) comprend une paire de parties allongées de bras (18, 19) qui sont insérées en position de cavalier sur le corps de la vis préalablement à la fixation finale de la tête de vis (4).

8. Système selon la revendication 7, **caractérisé en ce que** lesdites parties allongées de bras (18, 19) présentent des extrémités arrondies.

9. Système selon la revendication 5, **caractérisé en ce que** ledit élément plan intermédiaire (15) comprend une partie agrandie (21) ayant au moins une embase (22) destinée à retenir au moins une broche de fixation fragmentaire (23).

10. Système selon la revendication 9, **caractérisé en ce que** ladite embase (22) se présente sous la forme d'au moins un trou (22) réalisé dans ladite partie agrandie (21) de l'élément plan intermédiaire (15).

11. Système selon la revendication 9, **caractérisé en ce que** ladite embase (22) se présente sous la forme d'au moins un trou (24) réalisé dans au moins une des dites parties allongées de bras (18, 19).

12. Système selon la revendication 5, **caractérisé en ce que** ledit élément plan intermédiaire (15) présente un profil sensiblement arrondi.

13. Système selon la revendication 5, **caractérisé en ce que** ledit élément plan intermédiaire (15) est un joint ouvert formé solidaire avec une partie bombée (21).

14. Système selon la revendication 5, **caractérisé en ce qu'**un second élément plan intermédiaire (15') est inséré entre la tête de vis (4) d'une seconde vis de blocage (3), passant à travers un second trou proximal (6) du clou (10), et la surface du cortex de l'os (14).

15. Système selon la revendication 14, **caractérisé en ce que** ledit second élément plan intermédiaire (15') est plus grand qu'un premier élément de plaque intermédiaire (15).

16. Système selon la revendication 14, **caractérisé en ce que** ledit second élément plan intermédiaire (15') comprend une paire de parties allongées en bras (18, 19) qui sont insérées en position de cavalier sur le corps de la vis correspondante.
